# EUROPEAN PATENT APPLICATION

(11) **EP 4 851 632 A2**
(43) Date of publication of application: **09.12.2026**
(21) Application number: 26193231.3
(22) Date of filing: 31.10.2018
(51) Int. Cl.: G01N 33/68

(54) **ANTIBODY VARIANT AND ISOFORM WITH LOWERED BIOLOGICAL ACTIVITY**

(30) Priority: 01.11.2017 JP 2017212179
(62) Document number(s) of the earlier application(s) in accordance with Art. 76 EPC: 18873627.6 / 3 705 496
(71) Applicant: CHUGAI SEIYAKU KABUSHIKI KAISHA, Tokyo 115-8543 (JP)
(72) Inventor: HOSOGUCHI, Kensaku, Tokyo, 115-8543 (JP); KUWAYAMA, Maki, Tokyo, 115-8543 (JP); SEIDA, Chifumi, Tokyo, 115-8543 (JP); WATANABE, Yosuke, Tokyo, 115-8543 (JP); TANAKA, Nobuyuki, Tokyo, 115-8543 (JP); SAITOH, Satoshi, Tokyo, 115-8543 (JP); FUKUDA, Masakazu, Tokyo, 115-8543 (JP)
(74) Representative: Vossius & Partner Patentanwälte Rechtsanwälte mbB

(57) **Abstract**

As antibody variants and isoforms having reduced FVIII mimetic activity than Emicizumab, the antibody variants having some specific amino acid residues in the variable region cleaved out and missing (Q-CDR-Clipped Variants) and the antibody isoforms having inter-heavy chain disulfide bonds less susceptible to reduction under mild reducing conditions (Protected Disulfide Isoforms) are provided.

## Description

### [Technical Field]

The present invention relates to antibody variants and isoforms with reduced biological activity. For example, the present invention relates to antibody variants and isoforms of Emicizumab, the antibody variants and isoforms having reduced blood coagulation factor VIII (FVIII) mimetic activity. The present invention also relates to pharmaceutical compositions comprising such an antibody variant or isoform at low content rate. The present invention further relates to methods for detecting and methods for analyzing, for the antibody variants and isoforms.

### [Background Art]

Antibodies are drawing attention as pharmaceuticals as they are highly stable in plasma and have few adverse effects. Of them, a number of IgG-type antibody pharmaceuticals are available on the market and many antibody pharmaceuticals are currently under development (NPLs 1, 2 and 3).

Hemophilia A is a bleeding abnormality caused by a hereditary decrease or deficiency of FVIII function. Hemophilia A patients are generally administered with an FVIII formulation for bleeding (on-demand administration). In recent years, FVIII formulations are also administered prophylactically to prevent bleeding events (preventive administration; NPLs 1 and 2). The half-life of FVIII formulations in blood is approximately 12 to 16 hours. Therefore, for continuous prevention, FVIII formulations are administered to patients three times a week (NPLs 3 and 4). In on-demand administration, FVIII formulations are also additionally administered as necessary at an interval to prevent rebleeding. In addition, the administration of FVIII formulations is intravenous. Therefore, there has been a strong need for pharmaceutical agents with a lesser burden in administration than FVIII formulations.

Occasionally, antibodies against FVIII (inhibitors) are raised in hemophilia patients. Such inhibitors counteract the effects of the FVIII formulations. For bleeding in patients who have developed inhibitors (inhibitor patients), bypassing agents are administered. Their mechanisms of action do not depend on the FVIII function, that is, the function of catalyzing the activation of blood coagulation factor X (FX) by activated blood coagulation factor IX (FIXa). Therefore, in some cases, bypassing agents cannot sufficiently stop the bleeding. Accordingly, there has been a strong need for pharmaceutical agents that are not affected by the presence of inhibitors and which functionally substitute for FVIII.

As a means for solving the problem, bispecific antibodies that functionally substitute for FVIII and their use have been reported (PTLs 1, 2, 3, and 4). The bispecific antibodies against FIXa and FX can functionally substitute for FVIII by positioning the two factors close to each other to exhibit FVIII mimetic activity (NPL 5). It has been reported that the FVIII mimetic activity of the antibodies can be enhanced by optimizing the affinity towards FIXa and FX (NPL 6). Emicizumab (ACE910) having high FVIII mimetic activity, which is one of these antibodies, has been reported to exhibit hemostatic effects in monkey models of hemophilia (NPLs 7 and 8); therefore, clinical trials are being conducted on hemophilia A patients.

### [Citation List]

### [Patent Literature]

[PTL 1] WO 2005/035754
[PTL 2] WO 2005/035756
[PTL 3] WO 2006/109592
[PTL 4] WO 2012/067176

### [Non-Patent Literature]

[NPL 1] Blood 58, 1-13 (1981)
[NPL 2] Nature 312, 330-337(1984)
[NPL 3] Nature 312, 337-342(1984)
[NPL 4] Biochim.Biophys.Acta 871, 268-278(1986)
[NPL 5] Nat Med. 2012 Oct;18(10):1570-4.
[NPL 6] PLoS One. 2013;8(2):e57479.
[NPL 7] J Thromb Haemost. 2014 Feb;12(2):206-213.
[NPL 8] Blood. 2014 Nov 13;124(20):3165-71.
[NPL 9] J. Appl. Cryst. 13, 577-584 (1980)
[NPL 10] IUCrJ. 2, 9-18 (2015)

### [Summary of the Invention]

### [Technical Problem]

The present invention was achieved in view of the above circumstances. An objective of the present invention is to provide antibody variants or isoforms with reduced FVIII mimetic activity.

### [Solution to Problem]

The present inventors performed dedicated research to solve the above-described problems and succeeded in identifying antibody variants and isoforms which are contained in a pharmaceutical composition comprising Emicizumab as an active ingredient. The present inventors also found that FVIII mimetic activity of these antibody variants and isoforms is quite low, as compared with that of Emicizumab.

The present invention was made based on these findings, and provides [1] to [21] below.
[1] A variant of an antibody that comprises a variable region comprising the amino acid sequence SISPSGQSTYYRREVKG (SEQ ID NO: 2), wherein
   (a) the amino acid residue R at the 12th position from the N terminus side of the sequence (the 61th position from the N terminus side of the Emicizumab Q chain: position 60 according to Kabat numbering) or
   (b) the amino acid residues YYR at the 10th to 12th positions from the N terminus side of the sequence (the 59th to 61th positions from the N terminus side of the Emicizumab Q chain: positions 58 to 60 according to Kabat numbering)
   is deleted and the variable region is cleaved at the deletion site.
[2] The antibody variant of [1], wherein the sequence is a CDR sequence.
[3] The antibody variant of [1], wherein the sequence is a CDR2 sequence.
[4] The antibody variant of [1], wherein the sequence is a sequence comprised in a heavy chain.
[5] The antibody variant of [1], which is a variant of a bispecific antibody.
[6] The antibody variant of [1], which is a variant of Emicizumab.
[7] A method for detecting the antibody variant of any one of [1] to [6], comprising the step of separating a sample containing an antibody that comprises a variable region comprising the amino acid sequence SISPSGQSTYYRREVKG (SEQ ID NO: 2) by affinity chromatography, ion exchange chromatography, normal phase chromatography, reverse phase chromatography, hydrophilic interaction chromatography (HILIC), hydrophobic interaction chromatography (HIC), separation based on charge, size exclusion chromatography (SEC), gel permeation chromatography (GPC), or combinations thereof.
[8] The method for detecting of [7], which uses the antibody variant of any one of [1] to [6] as a reference standard.
[8-2] The method for detecting of [8], comprising the step of conducting one or more analysis selected from the group consisting of quantitative analysis, qualitative analysis, and structural analysis.
[9] A pharmaceutical composition comprising the antibody variant of any one of [1] to [6], wherein the percentage of the antibody variant in the total antibody molecules in the pharmaceutical composition is 5% or less.
[10] The pharmaceutical composition of [9], wherein the antibody is Emicizumab.
[11] The pharmaceutical composition of [9], which is obtained by a purification process comprising purification by cation exchange chromatography (CEX).
[12] A method for suppressing production of the antibody variant of any one of [1] to [6], comprising the step of culturing antibody producing cells at a pH of 7.1 or more, and/or at a culture temperature of 36°C or less.
[12-2] The method of [12], wherein conditions for culturing the antibody producing cells are changed to pH of 7.1 or more, and/or culture temperature of 36°C or less during culturing.
[13] An isoform of a bispecific antibody comprising a first heavy chain (Q chain, SEQ ID NO: 10) and a second heavy chain (J chain, SEQ ID NO: 11), wherein
   disulfide bonds are formed in the following:
   (1a) between cysteine at position 144 according to EU numbering of the first heavy chain (the 150th position from the N terminus side of SEQ ID NO: 10) and cysteine at position 200 according to EU numbering of the second heavy chain (the 202nd position from the N terminus side of SEQ ID NO: 11); and
   (1b) between cysteine at position 200 according to EU numbering of the first heavy chain (the 206th position from the N terminus side of SEQ ID NO: 10) and cysteine at position 144 according to EU numbering of the second heavy chain (the 146th position from the N terminus side of SEQ ID NO: 11), or wherein
   disulfide bonds are formed in the following:
   (2a) between cysteine at position 226 according to EU numbering of the first heavy chain (the 229th position from the N termunus side of SEQ ID NO: 10) and cysteine at position 229 according to EU numbering of the second heavy chain (the 228th position from the N terminus of SEQ ID NO: 11); and
   (2b) between cysteine at position 229 according to EU numbering of the first heavy chain (the 232nd position from the N terminus of SEQ ID NO: 10) and cysteine at position 226 according to EU numbering of the second heavy chain (the 225th position from the N terminus of SEQ ID NO: 11).
[14] The bispecific antibody isoform of [13], wherein disulfide bonds are formed in (1a) and (1b).
[15] An isoform of a bispecific antibody comprising a first heavy chain (Q chain, SEQ ID NO: 10) and a second heavy chain (J chain, SEQ ID NO: 11), characterized in that it elutes at a region more to the alkaline side than the bispecific antibody when separated using cation exchange chromatography.
[16] The bispecific antibody isoform of any one of [13] to [15], which is an isoform of Emicizumab.
[17] A method for detecting the antibody isoform of any one of [13] to [16], comprising the step of separating a sample containing a bispecific antibody by affinity chromatography, ion exchange chromatography, normal phase chromatography, reverse phase chromatography, hydrophilic interaction chromatography (HILIC), hydrophobic interaction chromatography (HIC), separation based on charge, size exclusion chromatography (SEC), gel permeation chromatography (GPC), or combinations thereof.
[18] The method for detecting of [17], which uses the bispecific antibody isoform of any one of [13] to [16] as a reference standard.
[18-2] The method for detecting of [18], comprising the step of conducting one or more analysis selected from the group consisting of quantitative analysis, qualitative analysis, and structural analysis.
[19] A pharmaceutical composition comprising the bispecific antibody isoform of any one of [13] to [16], wherein the percentage of the antibody isoform in the total antibody molecules in the pharmaceutical composition is 2% or less.
[20] A method for reducing the content percentage of the bispecific antibody isoform of any one of [13] to [16], comprising the step of purification by cation exchange chromatography.
[21] The antibody isoform or variant of [1], [13], or [15], wherein the biological activity of the antibody is markedly reduced.
[22] An isoform of an antibody having two variable regions each of which recognizes different epitopes or of a derivative thereof, wherein the isoform has an average Rg value that is smaller by 3% or more, preferably 4% or more, more preferably 5% or more, or still more preferably 6% or more, relative to the antibody or derivative thereof, and/or wherein the isoform has an average Dmax value that is smaller by 5% or more, preferably 6% or more, more preferably 7% or more, or still more preferably 7.5% or more, relative to the antibody or derivative thereof.
[23] An isoform of an antibody having two variable regions each of which recognizes different epitopes or of a derivative thereof, wherein the isoform has an average Rg value that is smaller by 0.15 nm or more, preferably 0.2 nm or more, more preferably 0.25 nm or more, or still more preferably 0.3 nm or more, relative to the antibody or derivative thereof, and/or wherein the isoform has an average Dmax value that is smaller by 0.5 nm or more, preferably 1.0 nm or more, more preferably 1.2 nm or more, or still more preferably 1.4 nm or more, relative to the antibody or derivative thereof.
[24] The isoform of [22] or [23], wherein the isoform has disulfide bond(s) different from those in the antibody or derivative thereof.
[25] The isoform of any one of [22] to [24], wherein the antibody or derivative thereof is Emicizumab (a bispecific antibody that comprises a first heavy chain (Q chain, SEQ ID NO: 10), a second heavy chain (J chain, SEQ ID NO: 11), and common light chains each one of which forms a pair with the first heavy chain or the second heavy chain (SEQ ID NO: 12)).
[26] An isoform of Emicizumab, wherein the isoform has an average Rg value of 4.9 nm or less, or preferably 4.8 nm or less, and/or wherein the isoform has an average Dmax value of 17.0 nm or less, or preferably 16.5 nm or less.
[27] The isoform of [25] or [26], wherein the isoform has disulfide bonds between cysteine at position 144 according to EU numbering of the first heavy chain (the 150th position from the N terminus side of SEQ ID NO: 10) and cysteine at position 200 according to EU numbering of the second heavy chain (the 202nd position from the N terminus side of SEQ ID NO: 11) and between cysteine at position 200 according to EU numbering of the first heavy chain (the 206th position from the N terminus side of SEQ ID NO: 10) and cysteine at position 144 according to EU numbering of the second heavy chain (the 146th position from the N terminus side of SEQ ID NO: 11).
[28] A pharmaceutical composition comprising Emicizumab and the isoform of any one of [22] to [27], wherein the percentage of the isoform in the total antibody molecules in the pharmaceutical composition is 2% or less.
[29] An isoform of a bispecific antibody (Q499-z121/J327-z119/L404-k; Emicizumab), the bispecific antibody comprising a first heavy chain (Q chain, SEQ ID NO: 10), second heavy chain (J chain, SEQ ID NO: 11), and common L chains each one of which forms a pair with the first heavy chain or the second heavy chain (SEQ ID NO: 12), wherein the isoform has a difference in molecular structure from Emicizumab in amino acid residues from position 146 according to EU numbering of the Q chain to position 174 according to EU numbering of the Q chain (the 152nd position to the 180th position from the N terminus side of SEQ ID NO: 10) and amino acid residues from position 146 according to EU numbering of the J chain to position 174 according to EU numbering of the J chain (the 148th position to the 176th position from the N terminus side of SEQ ID NO: 11).
[30] The isoform of [29], wherein the difference in molecular structure is measured as a difference in deuterium exchange rate (%D) in HDX-MS measurement.
[31] A pharmaceutical composition comprising Emicizumab and the isoform of [29] or [30], wherein the percentage of the isoform in the total antibody molecules in the pharmaceutical composition is 2% or less.

The present invention further provides [A1] to [A9] below.
[A1] The method for detecting of [7] or [17], comprising the step of subjecting a sample containing the antibody and/or the antibody variant or isoform to reduction reaction, hydrolysis reaction (digestion reaction), protein denaturation reaction, or a combination thereof.
[A2] The method for detecting of [A1], wherein the reduction reaction is carried out under mild reducing conditions (for example, reduction with DTT in Tris buffer (pH7.0) at 37°C).
[A3] The method for detecting of [A1], wherein the hydrolysis reaction is carried out using a site-specific cleavage enzyme (for example, a sequence-specific protease such as IdeS protease, Lys-C, papain, etc.).
[A4] The method for detecting of any one of [7], [17], and [A1] to [A3], comprising the step of separating a sample containing the antibody, the antibody variant or isoform, a reaction product thereof, or a combination thereof by affinity chromatography, ion exchange chromatography, normal phase chromatography, reverse phase chromatography, hydrophilic interaction chromatography (HILIC), hydrophobic interaction chromatography (HIC), separation based on charge, size exclusion chromatography (SEC), gel permeation chromatography (GPC), or combinations thereof.
[A5] The method for detecting of any one of [8], [18], and [A1] to [A4], comprising the step of analyzing by SE-HPLC analysis, dynamic light scattering (DLS) method, SAXS measurement, electron microscopic measurement, 3D modeling, SPR assay, HDX MS analysis, or a combination thereof.
[A6] A method for quality control of a pharmaceutical composition comprising Emicizumab, comprising the step of [7], [8], [17], [18], or [A1] to [A5] or a step of combining these methods.
[A7] A method for producing a pharmaceutical composition comprising Emicizumab, comprising a step from the method of [A6].
[A8] A method for purifying a composition comprising Emicizumab, characterized in that the method comprises a step of the Bind & Elute mode of cation exchange chromatography (CEX).
[A9] A method for producing a pharmaceutical composition comprising Emicizumab, comprising a step from the method for purifying of [A8].

### Effects of the Invention

The present inventors succeeded in identifying antibody variants and isoforms which are contained in a pharmaceutical composition comprising Emicizumab as an active ingredient. The present inventors also found that FVIII mimetic activity of these antibody variants and isoforms is quite low, as compared with that of Emicizumab. Therefore, pharmaceutical compositions comprising Emicizumab with such an antibody variant and isoform only at low content rate are useful as a means for treating hemophilia.

### [Brief Description of Drawings]

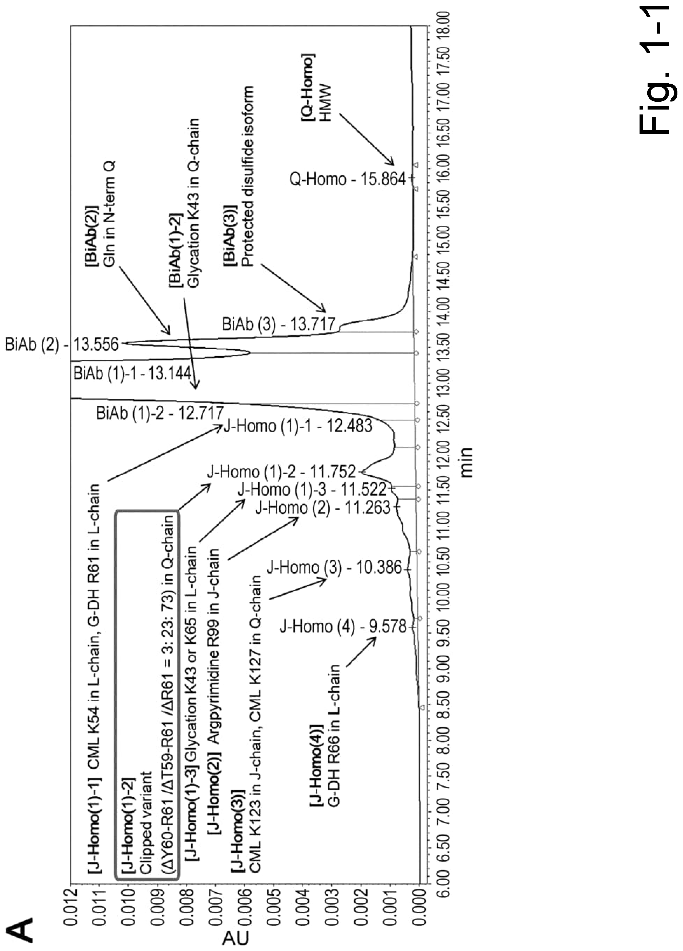

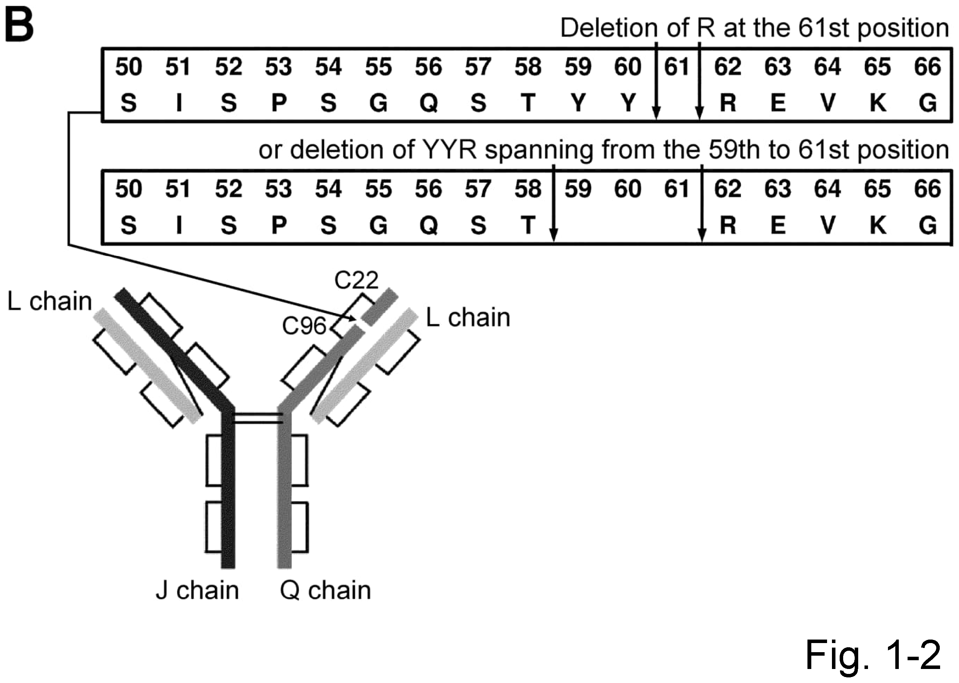

[Fig. 1-1] Fig. 1A shows the results of separation of Emicizumab drug substance by CE-HPLC. The peak marked with a thick-bordered box indicates Q-CDR-Clipped Variants.
[Fig. 1-2] Fig. 1B is a diagram showing the molecular structure of Q-CDR-Clipped Variants. The number and the alphabet letter just under it in the diagram indicate the position of an amino acid residue counted from the N-terminus of the Emicizumab Q chain and an amino acid residue (one-letter code) at that position, respectively.

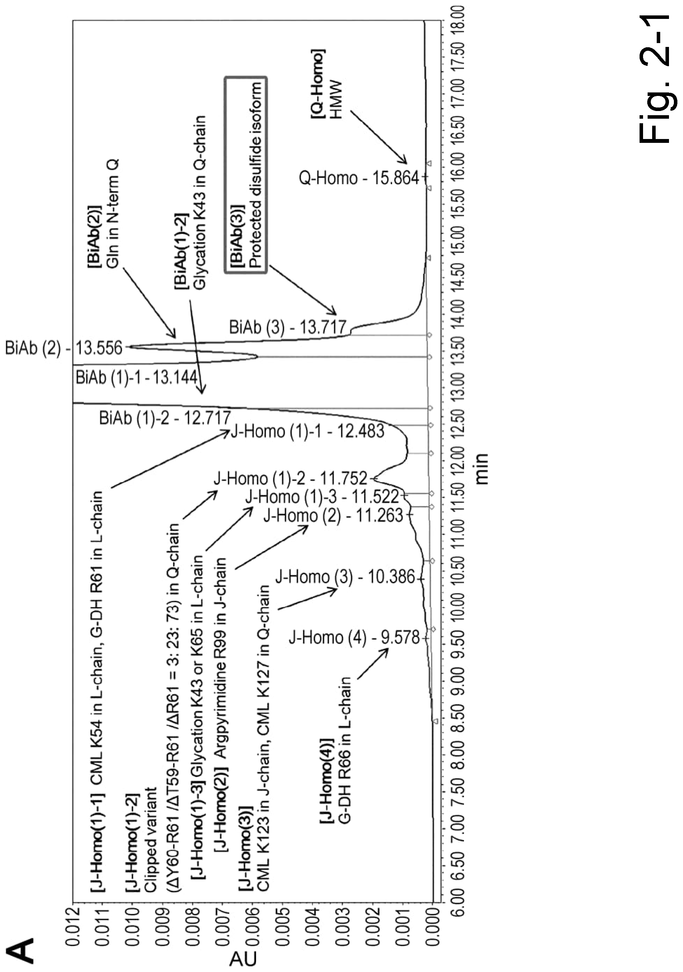

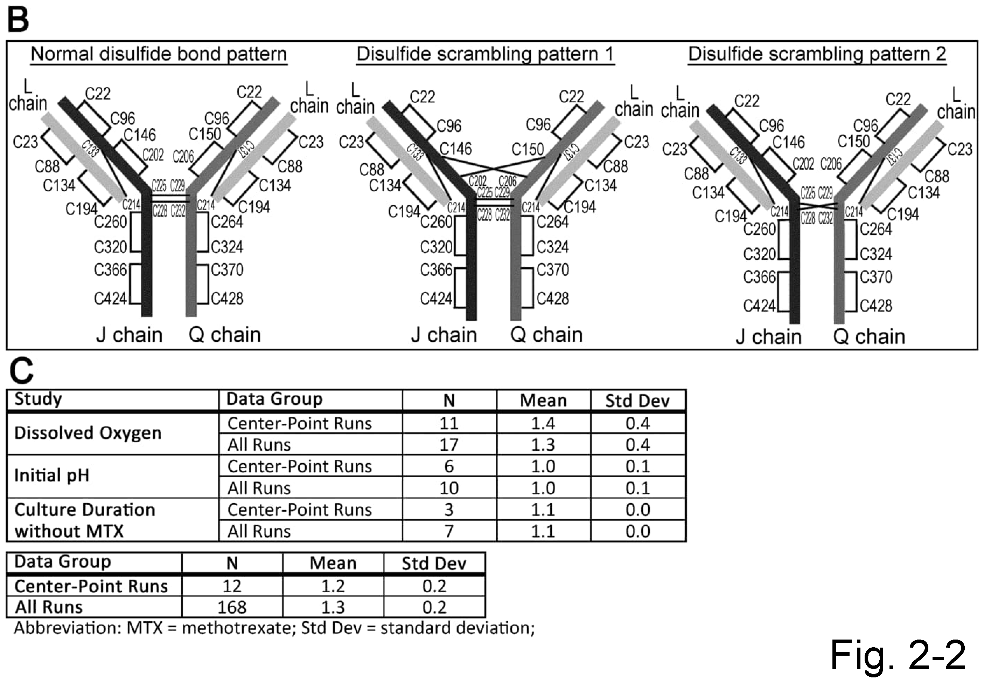

[Fig. 2-1] Fig. 2A shows the results of separation of Emicizumab drug substance by CE-HPLC. The peak marked with a thick-bordered box indicates Protected Disulfide Isoforms.
[Fig. 2-2] Fig. 2B is a diagram showing the molecular structure of Protected Disulfide Isoforms. The number and the alphabet letter C on the left of it in the diagram indicate the position of an amino acid residue counted from the N-terminus of the Emicizumab Q chain and a cysteine residue at that position, respectively. Fig. 2C shows the content rate of Protected Disulfide Isoforms in the Emicizumab drug substance. The content rates under various conditions (culture conditions for antibody-producing cells) are shown by the mean values (Mean) and their standard deviation (Std Dev) for the percentage of area (area %) of the peak of Protected Disulfide Isoforms in the CE-HPLC separation results, represented in Fig. 2A.

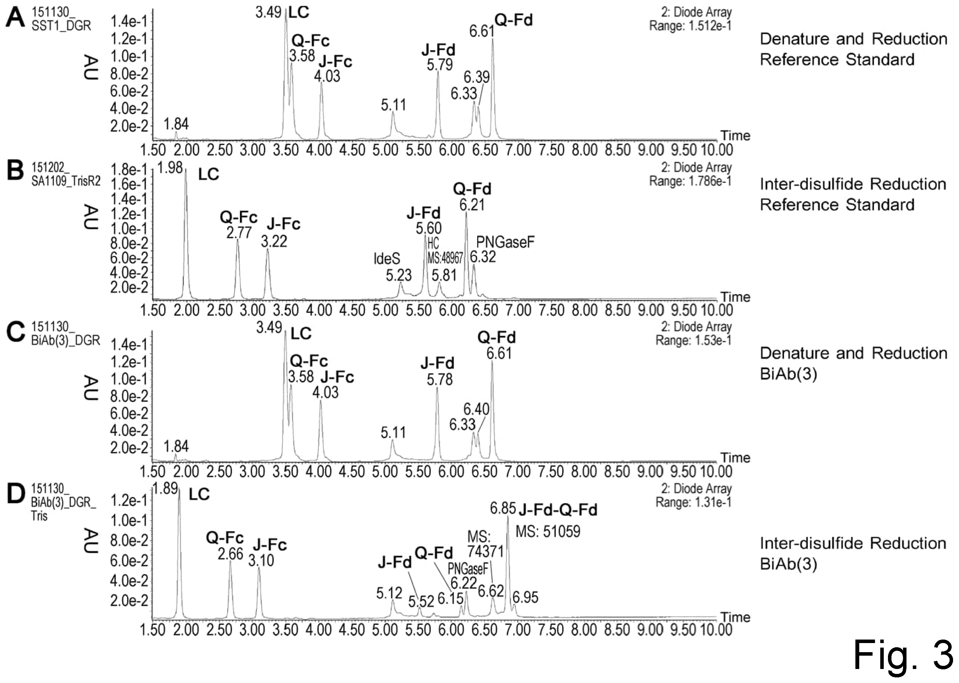

[Fig. 3] Fig. 3 shows the results of separating Emicizumab and Protected Disulfide Isoforms, after IdeS-digestion and reduction treatments, by reverse phase high performance liquid chromatography. Figs. 3A to D show the results of separation for samples containing Emicizumab (A and B) or Protected Disulfide Isoforms (C and D) which were IdeS digested and then reduced under the condition where the denaturant is present (A and C: complete reduction condition) or the condition where the denaturant is not present (B and D: partial reduction condition). For the samples from the full reduction condition (Figs. 3A and C), the peaks representing Q chain Fd (Q-Fd), J chain Fd (J-Fd), Q chain Fc (Q-Fc), J chain Fc (J-Fc), and L chain (LC) are all detected for Emicizumab and Protected Disulfide Isoforms: no difference in reduction pattern was detected between Emicizumab and Protected Disulfide Isoforms. On the other hand, for the samples from the partial reduction condition (Figs. 3B and D), the same peaks representing Q chain Fd, J chain Fd, Q chain Fc, J chain Fc, and L chain were detected as in the full reduction condition and, in addition thereto, a unique peak indicating the heterodimer of Q chain Fd and J chain Fd disulfide bonded together (J-Fd-Q-Fd) was detected only for Protected Disulfide Isoforms.

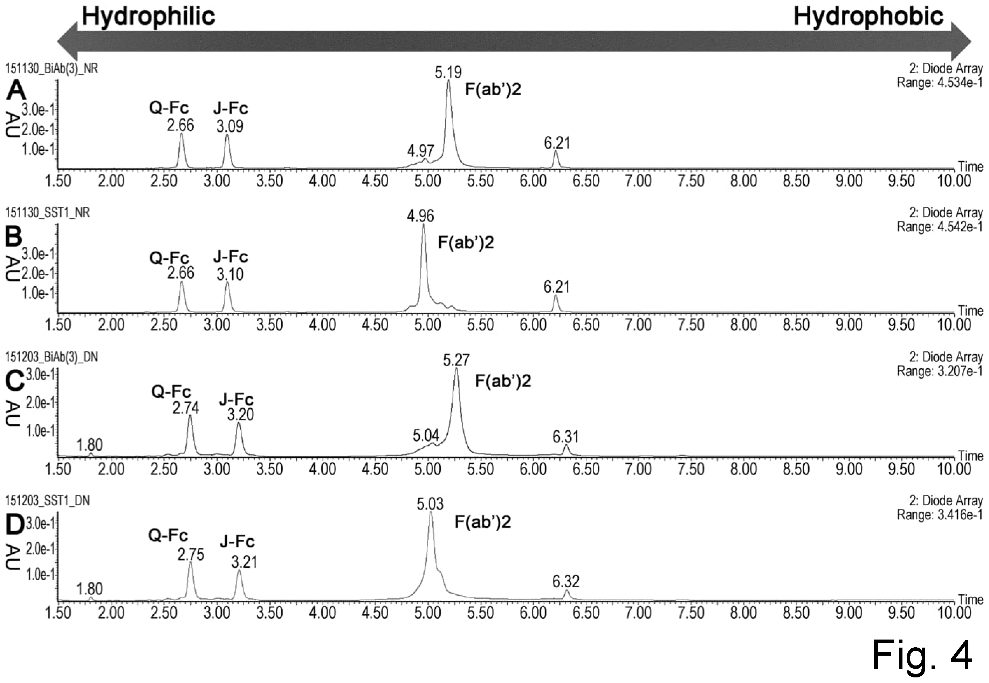

[Fig. 4] Fig. 4 shows the results of separating Emicizumab and Protected Disulfide Isoforms after IdeS digestion (and reduction treatment), by reverse phase high performance liquid chromatography. Figs. 4A and B show the results of separation for Protected Disulfide Isoforms (A) or Emicizumab (B) which were IdeS digested. Figs. 4C and D show the results of separation for Protected Disulfide Isoforms (C) or Emicizumab which were IdeS digested and then treated for denaturation. Regardless of whether being treated for denaturation or not, the F(ab')2 portion (LC-J Fab-Q Fab-LC) of Protected Disulfide Isoforms was separated with longer retention time than the main component of Emicizumab.

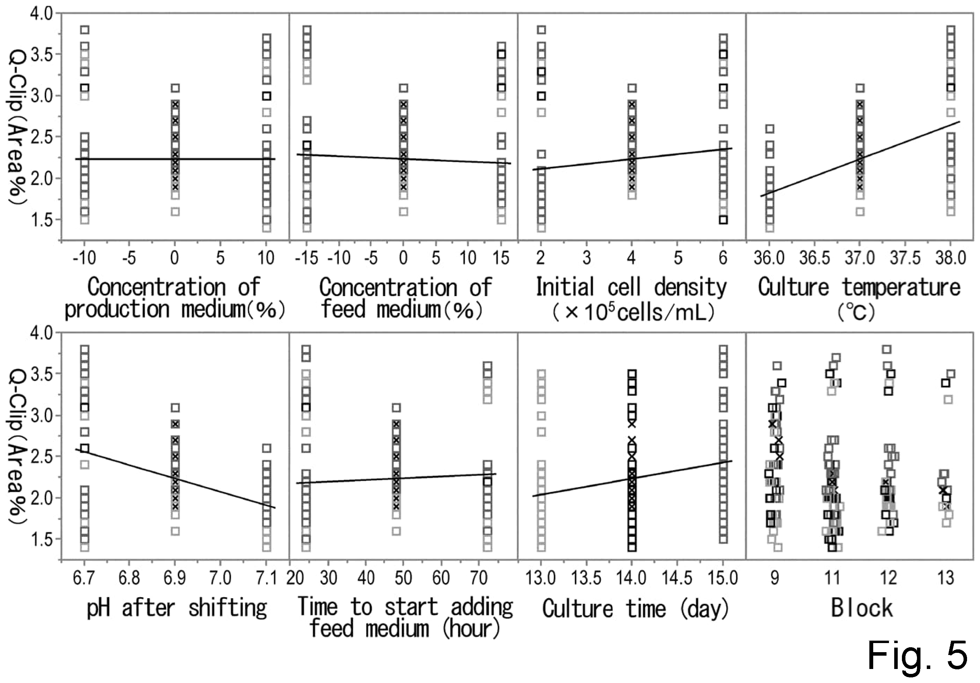

[Fig. 5] Fig. 5 shows the content rate of Q-CDR-Clipped Variants in culture supernatant of Emicizumab-producing CHO cells cultured under various culture conditions. Samples of culture supernatant purified using Protein A was used to measure the content rate of Q-CDR-Clipped Variants. The vertical axis indicates Q-CDR-Clipped Variant content rate (peak area %) and the horizontal axis indicates various culture conditions.

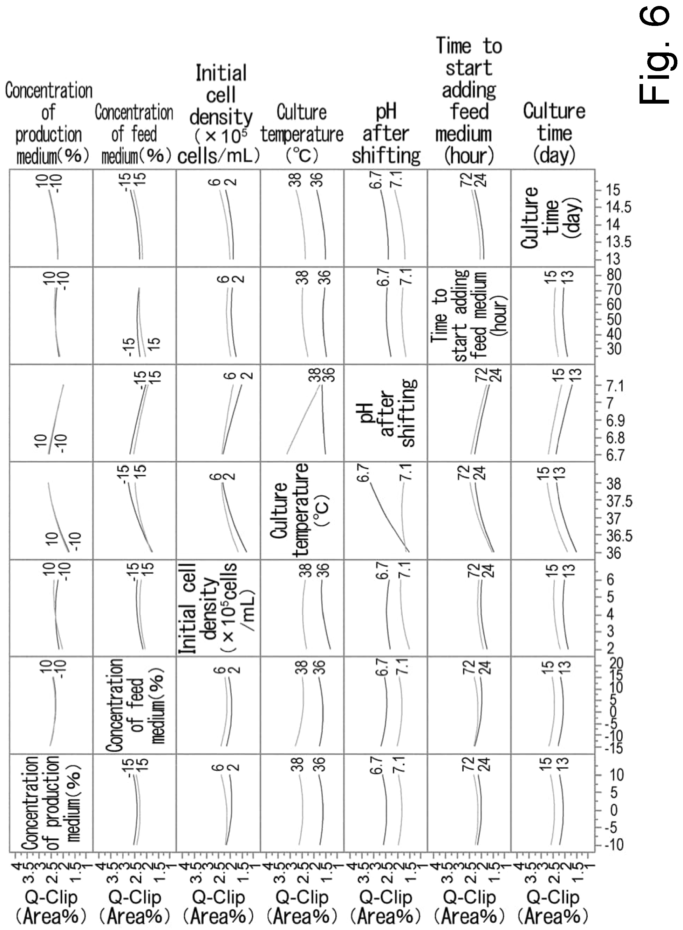

[Fig. 6] Fig. 6 shows the content rate of Q-CDR-Clipped Variants in culture supernatant of Emicizumab-producing CHO cells cultured under various culture conditions. Samples of culture supernatant purified using Protein A was used to measure the content rate of Q-CDR-Clipped Variants. The vertical axis indicates Q-CDR-Clipped Variant content rate (peak area %) and the horizontal axis indicates various culture conditions.

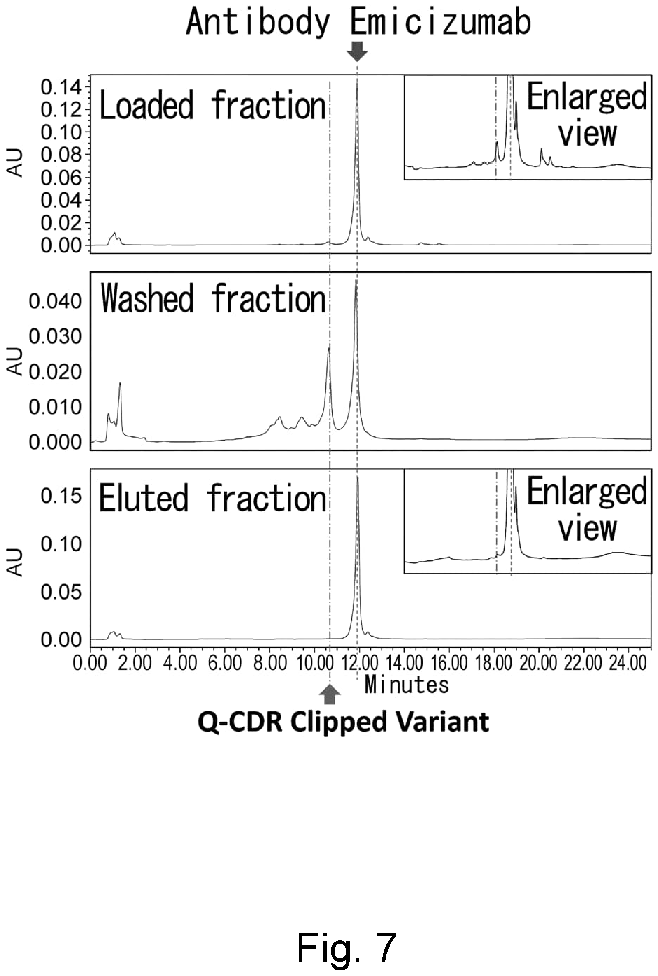

[Fig. 7] Fig. 7 shows the results of CE-HPLC analysis on each fraction of a solution of antibody Emicizumab containing Q-CDR-Clipped Variants, the fraction being obtained during purification including the steps in the Bind & Elute mode of cation exchange chromatography (CEX). "Loaded fraction" shows the results of the CE-HPLC analysis on the antibody solution loaded onto the cation exchange column. "Washed fraction" shows the results of the CE-HPLC analysis on the column-adsorbed fraction obtained after passing a pH 7.2 phosphate buffer containing 25 mmol/L sodium chloride (washed). "Eluted fraction" shows the results of the CE-HPLC analysis on the column-adsorbed fraction obtained after passing a pH 6.5 phosphate buffer containing 100 mmol/L sodium chloride. Compared with the loaded fraction and the washed fraction, the peak of Q-CDR-Clipped Variant on the more acidic side than the peak of antibody Emicizumab was found disappeared in the eluted fraction.

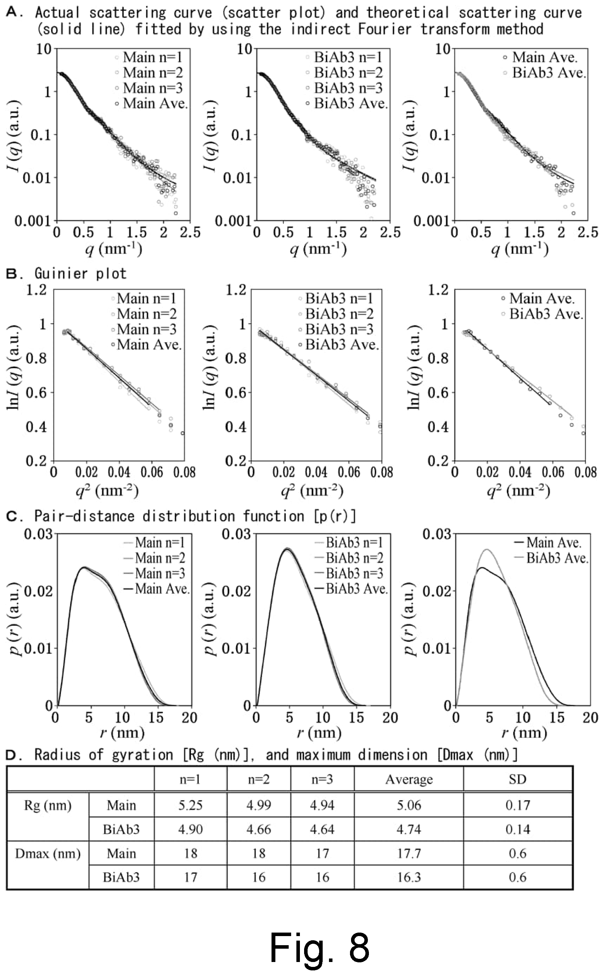

[Fig. 8] Fig. 8A-D show results from analyzing molecular structure of Emicizumab (Main) and Protected Disulfide Isoform (BiAb3) with the SAXS device. "Pair-distance distribution function [p(r)]", "Rg (nm)", and "Dmax (nm)" indicate the pair distance distribution function, radius of gyration, and maximum dimension, respectively.

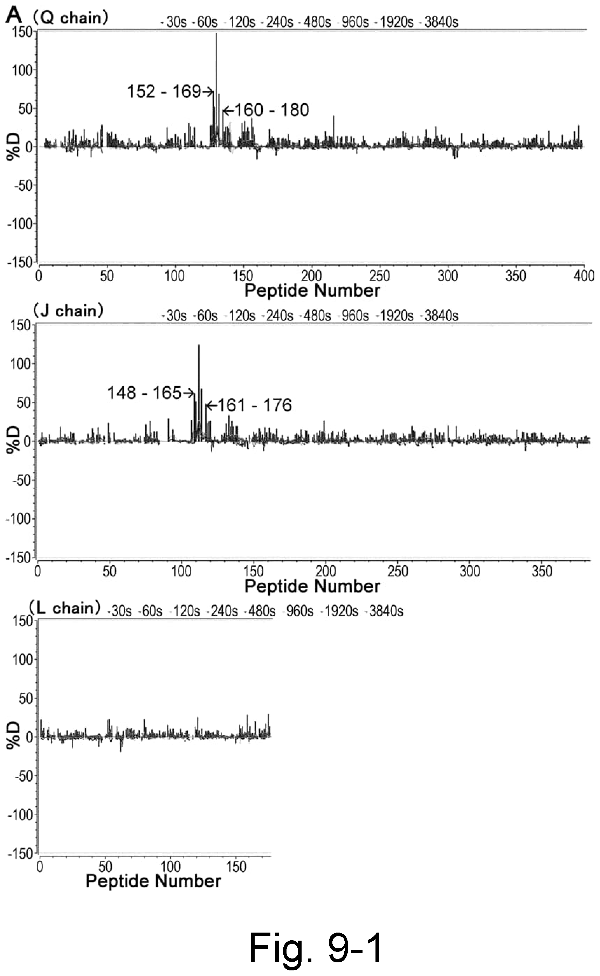

[Fig. 9-1] Fig. 9A shows residual plot of Emicizumab (main component in cation exchange high performance liquid chromatography) and the Protected Disulfide Isoforms on deuterium exchange rate (%D) in the HDX-MS measurement (deuterium exchange times 30 s, 60 s, 120 s, 240 s, 480 s, 960 s, 1920 s, and 3840 s). Each bar in the graph indicates the sum of the differences in the results from each deuterium exchange time for the Q chain, J chain, and L chain. Fig. 9B shows the parts for which difference in molecular structure in the Protected Disulfide Isoforms from Emicizumab was suggested by the HDX-MS measurement. Difference in molecular structure in the Protected Disulfide Isoforms from Emicizumab was notable in the peptide comprising the amino acid residues from position 146 according to EU numbering of the Q chain to position 174 according to EU numbering of the Q chain (the 152^{nd} position to the 180^{th} position from the N terminus side of SEQ ID NO: 10) and the peptide comprising the amino acid residues from position 146 according to EU numbering of the J chain to position 174 according to EU numbering of the J chain (the 148^{th} position to the 176^{th} position from the N terminus side of SEQ ID NO: 11). See the parts indicated with stars.

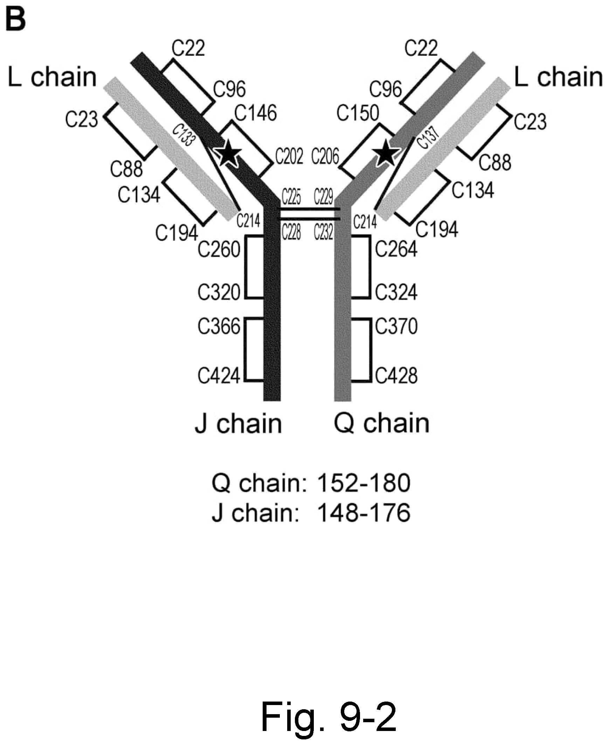

[Fig. 9-2] Fig. 9-2 is a continuation of Fig. 9-1.

### [Description of Embodiments]

One embodiment of the present invention relates to antibody variants and isoforms with reduced biological activity (for example, reduced FVIII mimetic activity). The antibody variants and isoforms were identified during the present inventors' analyzing the Emicizumab drug substance, as two types of structurally changed molecules (Q-CDR-Clipped Variants and the Protected Disulfide Isoforms). In the present application, the "antibody variant" and the "antibody isoform" may also be referred to as a mutant or isomer of an antibody molecule.

Emicizumab is a bispecific humanized IgG4 antibody showing an activity to functionally substitute for FVIII as cofactor and comprising an anti-FIX(a) and an anti-FX, and is composed of two types of heavy chains (Q499 and J327), each of which recognizes FIX(a) and FX respectively, and common L chains (L404).

Specifically, Emicizumab is a bispecific antibody where a first polypeptide and a third polypeptide form a pair, and a second polypeptide and a fourth polypeptide form a pair; where the first polypeptide comprises an H chain comprising the amino acid sequences of H-chain CDRs 1, 2, and 3 of SEQ ID NOs: 1, 2, and 3 (H-chain CDRs of Q499), respectively; the second polypeptide comprises an H chain comprising the amino acid sequences of H-chain CDRs 1, 2, and 3 of SEQ ID NOs: 4, 5, and 6 (H-chain CDRs of J327), respectively; and the third polypeptide and the fourth polypeptide comprise a common L chain comprising the amino acid sequences of L-chain CDRs 1, 2, and 3 of SEQ ID NOs: 7, 8, and 9 (L-chain CDRs of L404), respectively (Q499-z121/J327-z119/L404-k).

More specifically, Emicizumab is a bispecific antibody where a first polypeptide and a third polypeptide form a pair, and a second polypeptide and a fourth polypeptide form a pair; where the first polypeptide comprises an H chain comprising the amino acid sequence of H-chain variable region of SEQ ID NO: 13, the second polypeptide comprises an H chain comprising the amino acid sequence of H-chain variable region of SEQ ID NO: 14, and the third polypeptide and the fourth polypeptide comprise a common L chain comprising the amino acid sequence of L-chain variable region of SEQ ID NO: 15.

Still more specifically, Emicizumab is a bispecific antibody where a first polypeptide and a third polypeptide form a pair, and a second polypeptide and a fourth polypeptide form a pair; where the first polypeptide comprises an H chain comprising the amino acid sequence of SEQ ID NO: 10, the second polypeptide comprises an H chain comprising the amino acid sequence of SEQ ID NO: 11, and the third polypeptide and the fourth polypeptide comprise a common L chain of SEQ ID NO: 12 (Q499-z121/J327-z119/L404-k).

Such antibodies can be obtained by the methods described in WO 2005/035756, WO 2006/109592, WO 2012/067176, and such.

Antibodies used in the present invention are not particularly limited so long as they bind to a desired antigen, and they may be polyclonal or monoclonal antibodies. Monoclonal antibodies are preferred in that homogeneous antibodies can be stably produced.

Amino acids contained in the amino acid sequences of the present invention may be post-translationally modified (for example, the modification of an N-terminal glutamine into a pyroglutamic acid by pyroglutamylation is well-known to those skilled in the art). Naturally, such post-translationally modified amino acids are included in the antibodies used in the present invention.

In the present invention, the biological activity of the antibody or antibody variants or antibody isoforms is preferably FVIII mimetic activity. In the present invention the "FVIII mimetic activity" means an activity to functionally substitute for FVIII (activity to functionally substitute for FVIII as cofactor). In the present invention, the phrase "functionally substituting for FVIII" means recognizing FIX or FIXa, and FX, and promoting FX activation by FIXa (promoting FXa production by FIXa). FXa production-promoting activity can be evaluated using, for example, a measurement system comprising FIXa, FX, synthetic substrate S-2222 (synthetic substrate of FXa), and phospholipids. Such a measurement system shows a correlation with the disease severity and clinical symptoms in hemophilia A cases (Rosen S, Andersson M, Blombäck M et al. Clinical applications of a chromogenic substrate method for determination of FVIII activity. Thromb Haemost 1985; 54: 811-23).

FVIII mimetic activity of the antibodies such as Emicizumab and antibody variants and antibody isoforms can be evaluated, for example, methods described in WO 2005/035756, WO 2006/109592, WO 2012/067176, etc.

In the present invention, the antibodies or the antibody variants or isoforms are said to "have a reduced biological activity" when the biological activity is reduced as compared with the biological activity of a reference antibody, and it is preferred that the reduction is statistically significant. In the present invention, the antibodies or the antibody variants or the antibody isoforms are said to "have markedly (or extremely) reduced biological activity" when the biological activity is reduced as compared with the biological activity of a reference antibody by 10% or more, for example, 20% or more, 30% or more, 40% or more, 50% or more, 60% or more, 70% or more, 80% or more, or 90% or more.

In the present invention, the terms "Q chain" and "J chain" refer to an H chain (a heavy chain) comprising a variable region that can exhibit binding ability to FIX(a) and FX, respectively.

In the present invention, the term "common L chain" refers to an L chain that can form pairs with each of two or more different H chains and can exhibit binding ability to their respective antigen. Herein, the term "different H chains" preferably refers to H chains of antibodies against different antigens, but is not limited thereto; it refers to H chains whose amino acid sequences are different from each other. Common L chains can be obtained, for example, according to the methods described in WO 2006/109592.

The term "antibody" is used in the broadest sense, and includes monoclonal antibodies, polyclonal antibodies, dimers, multimers, multispecific antibodies (such as bispecific antibodies), antibody derivatives, and modified antibodies (Miller K et al. J Immunol. 2003, 170(9), 4854-61) so long as they show a desired biological activity. The antibodies may be mouse antibodies, human antibodies, humanized antibodies, chimeric antibodies, or those derived from another species, or artificially synthesized antibodies. The antibodies disclosed herein can be of any type (for example, IgG, IgE, IgM, IgD, and IgA), class (for example, IgGl, IgG2, IgG3, IgG4, IgAl and IgA2) or subclass of immunoglobulin molecules. The immunoglobulins can be derived from any species (for example, human, mouse, or rabbit). The terms "antibody", "immune globulin" and "immunoglobulin" are used interchangeably in a broad sense.

"Bispecific antibody" refers to an antibody having two variable regions that each recognize different epitopes, where the variable regions are present in the same antibody molecule. Bispecific antibodies may be antibodies that recognize two or more different antigens, or antibodies that recognize two or more different epitopes on the same antigen. Bispecific antibodies may include not only whole antibodies but antibody derivatives.

Recombinant antibodies produced by using genetic engineering techniques can be used as the antibodies. A recombinant antibody can be obtained by cloning a DNA encoding the antibody from hybridomas or antibody-producing cells such as sensitized lymphocytes that produce antibodies; inserting this into a vector; and then introducing it into hosts (host cells) to produce the antibody.

Bispecific antibodies are not limited to those of the IgG type; for example, IgG-type bispecific antibodies can be secreted from a hybrid hybridoma (quadroma) produced by fusing two types of hybridomas that produce IgG antibodies (Milstein C. et al., Nature 1983, 305: 537-540). They can also be secreted by introducing into cells the L-chain and H-chain genes constituting the two kinds of IgGs of interest, i.e., a total of four kinds of genes, to co-express the genes.

The antibodies of the present invention can be produced by methods known to those skilled in the art. Specifically, a DNA encoding the antibody of interest is inserted into an expression vector. The insertion into the expression vector is carried out such that the expression will take place under the control of expression regulatory regions such as an enhancer and a promoter. Next, host cells are transformed using this expression vector to express the antibody. Appropriate combinations of a host and an expression vector can be used in this case.

The antibodies of the present invention thus obtained can be isolated from the inside of host cells or the outside of the cells (medium, etc.), and purified to be substantially pure, homogeneous antibodies. The antibodies can be separated and purified by methods ordinarily used for separating and purifying antibodies, and the methods are not limited in any way. For example, methods described in WO 2013/086448 are known for separating IgG2 disulfide isoform. For separation and purification of the antibodies and the antibody variants or antibody isoforms in the present invention, for example, the antibodies and the antibody variants or antibody isoforms can be separated and purified by appropriately selecting and combining column chromatography, filtration, ultrafiltration, salting-out, solvent precipitation, solvent extraction, distillation, immunoprecipitation, SDS-polyacrylamide gel electrophoresis, isoelectrofocusing, dialysis, recrystallization, and such. For example, in the separation and purification using a chromatography column, various types of matrix including a strong cation exchange matrix, weak cation exchange matrix, anti-human IgG affinity matrix, and protein L matrix can be used.

In one aspect, the present invention relates to the antibody variants having the characteristic features described below (sometimes called "Q-CDR-Clipped Variants" in the present application):
- having extremely low biological activity (FVIII mimetic activity) as compared with Emicizumab;
- the fragment on the N-terminus side and the other fragment on the C-terminus side of the deleted amino acid residue(s) are joined together via a disulfide bond (Fig. 1B);
- the amount of production varies depending upon time, temperature, and pH for culturing antibody producing cells.

In one embodiment, Q-CDR-Clipped Variant is a variant of an antibody that comprises a variable region comprising the amino acid sequence SISPSGQSTYYRREVKG (SEQ ID NO: 2), wherein
(a) the amino acid residue R at the 12th position from the N terminus side of the amino acid sequence of SEQ ID NO: 2 (the 61th position from the N terminus side of the Emicizumab Q chain: position 60 according to Kabat numbering); or
(b) the amino acid residues YYR at the 10th to 12th positions from the N terminus side of the amino acid sequence of SEQ ID NO: 2 (the 59th to 61th positions from the N terminus side of the Emicizumab Q chain: positions 58 to 60 according to Kabat numbering),
is/are deleted and the variable region is cleaved at the deletion site.

Q-CDR-Clipped Variants are preferably variants of a bispecific antibody, and more preferably variants of Emicizumab.

In another aspect, the present invention relates also to methods for detecting and methods for analyzing Q-CDR-Clipped Variant. In one embodiment, the methods for detecting Q-CDR-Clipped Variant comprises the step of separating a sample containing an antibody that comprises a variable region comprising the amino acid sequence SISPSGQSTYYRREVKG (SEQ ID NO: 2) by affinity chromatography, ion exchange chromatography, normal phase chromatography, reverse phase chromatography, hydrophilic interaction chromatography (HILIC), hydrophobic interaction chromatography (HIC), separation based on charge, size exclusion chromatography (SEC), gel permeation chromatography (GPC), or a combination thereof. In one embodiment, the methods for analyzing Q-CDR-Clipped Variant comprise the step of conducting one or more analysis selected from the group consisting of quantitative analysis, qualitative analysis, and structural analysis, using a Q-CDR-Clipped Variant as a reference standard.

In such methods for detecting and methods for analyzing, the presence or absence of deleted part(s) in Fab having the amino acid sequence of SEQ ID NO: 2 (Q chain Fab) can be used as an indicator for the detection and analysis. The deleted part(s) may be detected, for example, by using a shift in the molecular weight resulting from the deletion in LCMS analysis as an indicator. In Q-CDR-Clipped Variants, the fragment on the N terminus side and the other fragment on the C-terminus side of the deleted amino acid residue(s) are joined together via a disulfide bond; therefore, difference in reduction patterns arising based on the presence or absence of the deleted part(s) can be detected by analyzing samples after being subjected to reaction to reduce disulfide bond(s), utilizing various analytical techniques such as CE-HPLC, LCMS, and LC-UV. Structural analysis by NMR measurement and such can also be utilized.

Alternatively, difference in resolution by ion exchange chromatography may be used as an indicator. For example, when separation is carried out by cation exchange chromatography, Q-CDR-Clipped Variants will be separated in a region more acidic than the main peak of Emicizumab.

In another aspect, in the present invention, production and quality control of pharmaceutical compositions comprising Emicizumab can be implemented by carrying out one of the above described methods for detecting and methods for analyzing or any combination thereof. Therefore, the present invention relates to methods for quality control of a pharmaceutical composition comprising Emicizumab, comprising a step of carrying out the above described methods for detecting and methods for analyzing or a step of combining any of those. The present invention also relates to production of a pharmaceutical composition comprising Emicizumab, comprising the step of carrying out such method(s) for quality control.

In another aspect, the present invention relates to a pharmaceutical composition comprising Emicizumab and Q-CDR-Clipped Variant(s), in which the ratio of Q-CDR-Clipped Variant(s) in total antibody molecules in the pharmaceutical composition is kept low. The pharmaceutical composition may be obtained by purification process comprising purification by cation exchange chromatography (CEX). For example, an antibody solution containing Emicizumab and Q-CDR-Clipped Variants is absorbed onto a cation exchange column, and after that, only acidic-side variants including Q-CDR-Clipped Variants may be selectively eluted and removed. The ratio of Q-CDR-Clipped Variants in total antibody molecules in the pharmaceutical composition can be evaluated by various methods, including the above-described methods for detecting/analyzing Q-CDR-Clipped Variant, and may be expressed by ratio of the peak area for Q-CDR-Clipped Variants (peak area ratio) from analyzing the pharmaceutical composition using cation exchange chromatography (CEX) or CE-HPLC, for example. The ratio of Q-CDR-Clipped Variants in total antibody molecules in the pharmaceutical composition (for example, CEX peak area ratio) is preferably 5% or less, and, for example, is 5.0% or less, 4.0% or less, 3.0% or less, 2.0% or less, or 1.0% or less.

The present invention further relates to methods for producing a pharmaceutical composition in which Q-CDR-Clipped Variant content rate is kept low, and methods for suppressing formation of Q-CDR-Clipped Variant. Q-CDR-Clipped Variant formation amount can be reduced by shortening culture time (for example, to 15 days or less, and preferably to 13 days or less), or by lowering culture temperature (for example, to 38°C or less, preferably to 37°C or less, and more preferably to 36°C or less), and/or by elevating culture pH (for example, to 6.7 or higher, preferably to 6.9 or higher, and more preferably to 7.1 or higher) for antibody-producing cells (Fig. 4). Therefore, the above-described methods are characterized in that the methods comprise the step of culturing antibody (for example, Emicizumab) producing cells at lower culture temperature (for example, about 36°C or less) and at higher pH (for example, about 7.1 or higher) than conventional, for a certain length of time (for example, about 15 days or less). In one embodiment, the above-described methods comprise the step of culturing antibody producing cells at pH of 7.1 or higher, and/or at culture temperature of 36°C or less. In certain embodiments, the above-described methods are characterized in that the culture condition for the antibody producing cells are shifted to at pH of 7.1 or higher and/or at culture temperature of 36°C or less in the midway of the culture (for example, on day 2 or later of the culture).

In another aspect, the present invention relates to methods for purifying a composition comprising Emicizumab, which methods are characterized in comprising a step of the Bind & Elute mode of cation exchange chromatography (CEX). The present invention further relates to methods for producing a pharmaceutical composition comprising Emicizumab, the methods comprising a step of carrying out the method for purification.

In another aspect, the present invention relates to the antibody isoforms having the characteristic features described below (sometimes called "Protected Disulfide Isoforms" in the present application):
- having an extremely low biological activity (FVIII mimetic activity) as compared with Emicizumab;
- more strongly hydrophobic as compared with Emicizumab;
- having inter-heavy chain disulfide bonds (Fig. 2B) which are less susceptible to reduction under mild conditions (partial reduction conditions) as compared with those in Emicizumab;
- formed irrespective of conditions (production parameters) such as dissolved oxygen concentration and initial pH of culture medium for antibody-producing cells, and culture time before adding MTX (methotrexate).

In one embodiment, Protected Disulfide Isoforms are characterized in that they can bind to FIX(a) and FX, the antigens, but do not exhibit biological activity (FVIII mimetic activity).

In certain embodiments, Protected Disulfide Isoforms are structural isomers having (normal) disulfide bonds identical to Emicizumab, but having stronger hydrophobicity than the usual due to structural change(s) in the Fab portion, thereby having inter-heavy chain disulfide bonds which have become less susceptible to reduction than the usual.

In another embodiment, Protected Disulfide Isoforms are isoforms of a bispecific antibody that comprises a first heavy chain (Q chain, SEQ ID NO: 10) and a second heavy chain (J chain, SEQ ID NO: 11), wherein, in Protected Disulfide Isoforms,
disulfide bonds are formed in the following:
(1a) between cysteine at position 144 according to EU numbering of the first heavy chain (the 150th position from the N terminus side of SEQ ID NO: 10) and cysteine at position 200 according to EU numbering of the second heavy chain (the 202nd position from the N terminus side of SEQ ID NO: 11); and
(1b) between cysteine at position 200 according to EU numbering of the first heavy chain (the 206th position from the N terminus side of SEQ ID NO: 10) and cysteine at position 144 according to EU numbering of the second heavy chain (the 146th position from the N terminus side of SEQ ID NO: 11), or wherein
disulfide bonds are formed in the following:
(2a) between cysteine at position 226 according to EU numbering of the first heavy chain (the 229th position from the N terminus side of SEQ ID NO: 10) and cysteine at position 229 according to EU numbering of the second heavy chain (the 228th position from the N terminus side of SEQ ID NO: 11); and
(2b) between cysteine at position 229 according to EU numbering of the first heavy chain (the 232nd position from the N terminus side of SEQ ID NO: 10) and cysteine at position 226 according to EU numbering of the second heavy chain (the 225th position from the N terminus side of SEQ ID NO: 11).

In a particular embodiment, Protected Disulfide Isoforms are isoforms of a bispecific antibody and preferably are isoforms of Emicizumab, wherein, in Protected Disulfide Isoforms, disulfide bonds are formed in the following:
(1a) between cysteine at position 144 according to EU numbering of the first heavy chain (the 150th position from the N terminus side of SEQ ID NO: 10) and cysteine at position 200 according to EU numbering of the second heavy chain (the 202nd position from the N terminus side of SEQ ID NO: 11);
(1b) between cysteine at position 200 according to EU numbering of the first heavy chain (the 206th position from the N terminus side of SEQ ID NO: 10) and cysteine at position 144 according to EU numbering of the second heavy chain (the 146th position from the N terminus side of SEQ ID NO: 11);
(1c) between cysteine at position 226 according to EU numbering of the first heavy chain (the 229th position from the N terminus side of SEQ ID NO: 10) and cysteine at position 226 according to EU numbering of the second heavy chain (the 225th position from the N terminus side of SEQ ID NO: 11); and
(1d) between cysteine at position 229 according to EU numbering in the first heavy chain (the 232nd position from the N terminus side of SEQ ID NO: 10) and cysteine at position 229 according to EU numbering in the second heavy chain (the 228th position from the N terminus side of SEQ ID NO: 11), or wherein
disulfide bonds are formed in the following:
(2a) between cysteine at position 226 according to EU numbering of the first heavy chain (the 229th position from the N terminus side of SEQ ID NO: 10) and cysteine at position 229 according to EU numbering of the second heavy chain (the 228th position from the N terminus side of SEQ ID NO: 11);
(2b) between cysteine at position 229 according to EU numbering of the first heavy chain (the 232nd position from the N terminus side of SEQ ID NO: 10) and cysteine at position 226 according to EU numbering of the second heavy chain (the 225th position from the N terminus side of SEQ ID NO: 11);
(2c) between cysteine at position 144 according to EU numbering of the first heavy chain (the 150th position from the N terminus side of SEQ ID NO: 10) and cysteine at position 200 according to EU numbering of the first heavy chain (the 206th position from the N terminus side of SEQ ID NO: 10); and
(2d) between cysteine at position 144 according to EU numbering of the second heavy chain (the 146th position from the N terminus side of SEQ ID NO: 11) and cysteine at position 200 according to EU numbering of the second heavy chain (the 202nd position from the N terminus side of SEQ ID NO: 11).

In another aspect, the present invention relates to methods for detecting and methods for analyzing Protected Disulfide Isoform. In one embodiment, the methods for detecting Protected Disulfide Isoform comprises the step of separating a sample containing a bispecific antibody by affinity chromatography, ion exchange chromatography, normal phase chromatography, reverse phase chromatography, hydrophilic interaction chromatography (HILIC), hydrophobic interaction chromatography (HIC), separation based on charge, size exclusion chromatography (SEC), gel permeation chromatography (GPC), or a combination thereof. In one embodiment, the methods for analyzing Protected Disulfide Isoform comprise the step of conducting one or more analysis selected from the group consisting of quantitative analysis, qualitative analysis, and structural analysis, using Protected Disulfide Isoform as a reference standard.

In such methods for detecting and methods for analyzing, analysis can be made utilizing difference(s) between Protected Disulfide Isoforms and Emicizumab in the structure of the region(s) forming inter-heavy chain disulfide bonds and/or of the Fab region. The structural difference(s) can be detected by various analytical methods, such as those shown below for example.

For example, the peak(s) reflecting the difference in three dimensional structure or strength of hydrophobicity between Emicizumab and Protected Disulfide Isoforms can be detected by analyzing a sample using a reverse phase column (for example, a C4 column), after the sample has been treated for digestion reaction by IdeS protease under non-reducing conditions (to cleave at a single site below the hinge region in IgG to give F(ab')2 and Fc fragments).

The peak(s) reflecting the difference in disulfide bond's susceptibility to reduction between Emicizumab and Protected Disulfide Isoforms can be detected by analyzing a sample using a reverse phase column, after the sample has been treated for IdeS digestion and thereafter for reduction reaction under mild reducing conditions (for example, reduction with DTT in Tris buffer solution (pH7.0) at 37°C).

No difference will be detected in analysis results obtained using a reverse phase column for Emicizumab and Protected Disulfide Isoforms when the reduction reaction is carried out under the conditions where all disulfide bonds are reduced, whereas difference in reduction patterns will be detected in analysis results obtained using a reverse phase column for them when the reduction reaction is carried out under the above-described mild conditions. Without wishing to be limited by any particular theory, it is thought that, under the above-described mild reducing conditions, the disulfide bonds between the heavy chain and the light chain and the disulfide bonds between the heavy chains are all reduced in the case of Emicizumab, whereas only the disulfide bonds between the heavy chain and the light chain are reduced and the disulfide bonds between the heavy chains are left unreduced, in the case of Protected Disulfide Isoforms.

The peak(s) reflecting the difference in the Lys-C digestion pattern arising from the difference in three-dimensional structure between Emicizumab and Protected Disulfide Isoforms can be detected by analyzing a sample using a reverse phase column (for example, a C4 column), after the sample has been treated for limited Lys-C digestion (for example by stopping Lys-C digestion reaction halfway) under non-denaturing conditions (for example, in a Tris buffer solution). Without wishing to be limited by any particular theory, it is thought that the Lys-C digestion patterns reflecting the difference in three-dimensional structure are detected, as a result that cleavage is made preferentially at positions where Lys-C is easily accessible in a state where three-dimensional structure is retained during Lys-C digestion under non-denaturing conditions.

Alternatively, the peak(s) reflecting the difference in the Lys-C digestion pattern arising from the difference in three-dimensional structure between Emicizumab and Protected Disulfide Isoforms can be detected by analyzing a sample using a reverse phase column, after the sample, pre-treated for denaturation (for example, treated with 5M guanidine at 37°C for 30 minutes) but not reduced (i.e., retains the SS bonds), has been treated for limited Lys-C digestion. Without wishing to be limited by any particular theory, it is thought that the Lys-C digestion patterns reflecting the difference in the SS bonds are detected, as a result of that cleavage is made preferentially at positions where Lys-C is easily accessible in a state where three-dimensional structure is no longer retained but the SS bonds (disulfide bonds) are retained as the Lys-C digestion is carried out for the denatured but non-reduced samples.

In addition to the above-described reduction reaction, IdeS digestion, and limited Lys-C digestion under non-denaturing conditions or denaturing conditions, various other decomposition reactions such as papain digestion can be utilized. In addition to reverse phase chromatography using a C4 column and such, various analytical techniques such as SE-HPLC analysis, dynamic light scattering (DLS), SAXS measurement, electron microscopy measurement, 3D modeling, SPR assay, HDX MS analysis can be utilized.

In another aspect, in the present invention, production and quality control of pharmaceutical compositions comprising Emicizumab can be implemented by carrying out one of the above described methods for detecting and methods for analyzing or any combination thereof. Therefore, the present invention relates to methods for quality control of a pharmaceutical composition comprising Emicizumab, comprising a step of carrying out the above described methods for detecting and methods for analyzing or a step of combining any of those. The present invention also relates to production of a pharmaceutical composition comprising Emicizumab, comprising the step of carrying out such method(s) for quality control.

In another aspect, the present invention relates to a pharmaceutical composition comprising Emicizumab and Protected Disulfide Isoform(s), in which the ratio of Protected Disulfide Isoform(s) in total antibody molecules in the pharmaceutical composition is kept low. The ratio of Protected Disulfide Isoform in total antibody molecules in the pharmaceutical composition can be evaluated by various methods, including the above-described methods for detecting/analyzing Protected Disulfide Isoform, and may be expressed by ratio of the peak area for Protected Disulfide Isoform (peak area ratio) from analyzing the pharmaceutical composition using cation exchange chromatography (CEX) or CE-HPLC, for example. The ratio of Protected Disulfide Isoform in total antibody molecules in the pharmaceutical composition (for example, CEX peak area ratio) is preferably 2% or less, and, for example, is 2.0% or less, 1.5% or less, 1.0% or less, or 0.5% or less.

In another aspect, the present invention relates to methods for purifying a composition comprising Emicizumab, which methods are characterized in comprising a step of the Bind & Elute mode of cation exchange chromatography (CEX). The present invention further relates to methods for producing a pharmaceutical composition comprising Emicizumab, the methods comprising a step of carrying out the method for purification.

In another aspect, the present invention discloses isoforms of Emicizumab (Protected Disulfide Isoforms) having the same heavy chain and light chain amino acid sequences with those of Emicizumab but having a molecular structure with smaller Rg (nm) value and/or Dmax (nm) value than Emicizumab. Such isoforms have a molecular structure with shorter distance between the J-chain/Q-chain N termini than Emicizumab, and specifically have an average Rg value measured with the SAXS device that is smaller by 3% or more, preferably 4% or more, more preferably 5% or more, or still more preferably 6% or more, relative to Emicizumab, and/or have an average Dmax value measured with the SAXS device that is smaller by 5% or more, preferably 6% or more, more preferably 7% or more, or still more preferably 7.5% or more, relative to Emicizumab. The isoforms may have an average Rg value that is smaller by 0.15 nm or more, preferably 0.2 nm or more, more preferably 0.25 nm or more, or still more preferably 0.3 nm or more, relative to Emicizumab, and/or have an average Dmax value that is smaller by 0.5 nm or more, preferably 1.0 nm or more, more preferably 1.2 nm or more, or still more preferably 1.4 nm or more, relative to Emicizumab. These isoforms may have an average Rg value of 4.9 nm or less, or preferably 4.8 nm or less, and/or have an average Dmax value of 17.0 nm or less, or preferably 16.5 nm or less.

The Rg and Dmax values can be measured under the conditions identified below:
(1) Antibody concentration: an antibody concentration of 7.54 mg/mL;
(2) Solvent conditions: 150 mmol/L arginine, 20 mmol/L histidine-aspartic acid, pH6.0; and
(3) Temperature: 25°C.

Here, an average Rg value can be obtained by calculating Rg for each measurement from Guinier plot and by taking an average over them. An average Dmax value can be obtained by calculating Dmax for each measurement from x-intercept of p(r) and by taking an average over them. For methods of analyzing Guinier plot and p(r), see Example 9 described below.

In another aspect, the present invention discloses isoforms of Emicizumab (Protected Disulfide Isoforms) having the same heavy chain and light chain amino acid sequences with those of Emicizumab but having a different molecular structure as compared with Emicizumab in the amino acid residues from position 146 according to EU numbering of the Q chain to position 174 according to EU numbering of the Q chain (the 152^{nd} position to the 180^{th} position from the N terminus side of SEQ ID NO: 10) and the amino acid residues from position 146 according to EU numbering of the J chain to position 174 according to EU numbering of the J chain (the 148^{th} position to the 176^{th} position from the N terminus side of SEQ ID NO: 11). The difference in molecular structure may be measured as a difference in deuterium exchange rate (%D) in HDX-MS measurement, and may be confirmed as a difference in deuterium exchange times for the peptides comprising the amino acid residues of these regions, as specifically shown in Fig. 9A.

In another aspect, the present invention discloses pharmaceutical compositions comprising Emicizumab and the isoform, wherein the percentage of the isoform in the total antibody molecules in the pharmaceutical composition is 2% or less.

In the above-described antibody molecules, antibody variants, antibody isoforms, pharmaceutical compositions comprising an antibody variant or isoform, methods for analyzing an antibody variant or isoform, or methods for suppressing formation of an antibody variant or isoform, the antibody is preferably a bispecific antibody and more preferably is Emicizumab (ACE910).

As used herein, aspects referred to by the expression "comprising" include those referred to by the expression "essentially consisting of", and those referred to by the expression "consisting of".

Numerical values recited herein may vary within a certain range, for example, depending on the instruments or equipment, measurement conditions, and procedure used by those skilled in the art, and so long as they are within a range that allows the objective of the invention to be accomplished, they may encompass a deviation of approximately 10%, for example.

All patents and references explicitly cited herein are incorporated by reference into this specification in its entirety.

The present invention will be further illustrated by the Examples below, but it is not to be construed as being limited thereto.

### [Examples]

### [Example 1] Preparation of a genetically-engineered humanized bispecific monoclonal antibody (antibody Emicizumab)

For structural analysis of Emicizumab isomers (antibody variants and isoforms), antibody Emicizumab was produced in a large amount by the method described below. CHO cells into which a gene encoding Emicizumab was introduced were cultured as Emicizumab producing cells in a commercially available basal medium (basal medium for culturing animal cells). Culture was conducted under conditions giving environment generally suitable for culturing CHO cells.

Expressed antibody was purified by combination of standard column chromatography, such as affinity chromatography, ion-exchange chromatography, hydrophobic chromatography, etc.

### [Example 2] Separation of Q-CDR-Clipped Variants and Protected Disulfide Isoforms (cation exchange high performance liquid chromatography)

The sample solution, prepared by diluting a specimen to analyze with mobile phase solution A (composition thereof is described below), was poured into a cation exchange column (ProPac WCX-10: particle diameter, 10 µm; inner diameter, 4.0 mm, length, 250 mm). Then, separation was carried out by liquid chromatography (column temperature: 30+/- 5°C, measurement wavelength: 280 nm, flow rate: 1.0 mL/min) using an acidic mobile phase (mobile phase solution A containing 9.6 mmol/L Tris, 6.0 mmol/L piperazine, 11.0 mmol/L imidazole buffer (pH 6.0)) and an alkaline mobile phase (mobile phase solution B containing 9.6 mmol/L Tris, 6.0 mmol/L piperazine, 11.0 mmol/L imidazole, and 150 mmol/L sodium chloride (pH 9.9)). As a result, it was confirmed that Q-CDR-Clipped Variants and Protected Disulfide Isoforms were separated in more acidic and alkaline regions, respectively, as compared with the main peak corresponding to Emicizumab (Fig. 1A and Fig. 2A).

### [Example 3] Separation of Protected Disulfide Isoforms (IdeS digestion, partial reduction, reverse phase high performance liquid chromatography)

Specimens were diluted using phosphate buffer, digested with IdeS protease and PNGase-F, and then partially reduced with DTT in Tris buffer solution containing no denaturant. The sample diluted using TFA solution was poured into the reverse phase high performance liquid chromatography column and separated. As a result, it was found that the main component of Emicizumab is separated on a chromatograph in the state where the disulfide bonds between the heavy and light chains are reduced, whereas Protected Disulfide Isoforms are detected with unique peaks representing the state where the disulfide bonds between the heavy chains remain not reduced (Fig. 3B and Fig. 3D).

### [Example 4] Separation of Protected Disulfide Isoforms (IdeS digestion, denaturation, reverse phase high performance liquid chromatography)

Specimens were diluted with a buffer, digested with IdeS protease and PNGase-F, and then proteins were denatured using a denaturation buffer. After that, the sample diluted using TFA solution was poured into the reverse phase high-performance liquid chromatography column and separated. As a result, it was found that the F(ab')2 portion of Protected Disulfide Isoforms is separated after a longer retention time than the main component of Emicizumab (Fig. 4C and Fig. 4D).

### [Example 5] Separation of Protected Disulfide Isoforms (IdeS digestion, reverse phase high performance liquid chromatography)

Specimens were diluted using a buffer, and digested with IdeS protease and PNGase-F. The sample diluted using TFA solution was separated by reverse phase high performance liquid chromatography. As a result, it was found that the F(ab')2 portion of Protected Disulfide Isoforms is separated after a longer retention time than the main component of Emicizumab (Fig. 4A and Fig. 4B).

### [Example 6] Evaluation of biological activity of Q-CDR-Clipped Variants and Protected Disulfide Isoforms

### Chromogenic assay

The amount of activated blood coagulation factor X (FXa), produced through reaction of Emicizumab in the presence of FIXa and FX in a reaction field where phospholipid was supplied, was quantitatively measured using a specific chromogenic substrate. Specifically, Emicizumab solutions diluted at various concentrations were prepared by adding a solution (TBSB) containing Tris-hydroxymethyl aminomethane, sodium chloride, and BSA to a sample. Each diluted solution was added to its respective well in a 96-well microplate, a coagulation factor solution containing FIXa, FX, calcium chloride, magnesium chloride, phospholipid, and TBSB was added thereto, and, after shaking, the plate was left for 30 minutes. Ethylene diamine tetraacetic acid solution was added to each well, the plate was shaken, and a chromogenic substrate solution was added to each well (N-benzoyl-L-isoleucyl-L-glutamyl-glycyl-L-arginine-p-nitroaniline hydrochloride and its methyl ester). After shaking, the plate was left for 35 minutes. After one to two minutes of shaking, absorbance (Abs) at 405 nm was measured for each well using a plate reader. Based on the absorbance values obtained from standard solutions and sample solutions at various concentrations, specific activity of the samples relative to the standard solutions were determined from a regression curve generated using 4-parameter-parallel-lines-logistics analysis program. As a result, Q-CDR-Clipped Variants exhibited 18 +/-1%, Protected Disulfide Isoforms exhibited 8 +/- 0% of biological activity relative to the Emicizumab standard solution.

### Clotting assay

In this assay, the time until clotting due to fibrin formation was measured based on changes in turbidity as indicator, in a system reconstituting endogenous coagulation activation mechanism using factor VIII deficient human plasma. Specifically, a solution containing Tris-hydroxymethyl aminomethane, sodium chloride, and BSA was added to the sample to prepare Emicizumab solutions diluted at various concentrations. Using an automatic blood coagulation measuring device, the diluted solutions were added with factor VIII deficient plasma and incubated, then were added with APTT reagent and incubated, and lastly were added with calcium chloride solution and measured to determine the clotting time. The specific activity of the samples relative to the standard substance was calculated by parallel line assay. As a result, Q-CDR-Clipped Variants exhibited 18 +/- 1%, Protected Disulfide Isoforms exhibited 16 +/- 1% of biological activity relative to the Emicizumab standard solution.

### [Example 7] Assessment of effect of culture parameters on the ratio of Q-CDR-Clipped Variants Initial culture medium

Plant-derived hydrolyzates, amino acids, and such were added and dissolved into commercially available basal medium. The mixture was then sterilized by filtration.

### Feed medium

Glucose, amino acids, and such were added and dissolved into commercially available basal medium. The mixture was then sterilized by filtration.

### Cells

Emicizumab producing CHO cells (DXB-11 strain) comprising a gene encoding Emicizumab incorporated therein were used.

### Culture method

Production medium (+/-10% to the standard concentration) was poured into a 1L-scale cell culture device and the above-described CHO cell strain was seeded thereto to give 2 to 6 x 10⁵ cells/mL. Cell culture was started at the temperature of 36 to 38°C, dissolved oxygen concentration of 40%, initial pH of 7.20. Starting from day 1 to 3 of culture, feed medium (+/-10% to the standard concentration) was added at a constant flow rate, and on day 3 of culture, pH was shifted to 6.70-7.10. Culture was continued for 13 to 15 days.

Culture was conducted under a total of 56 conditions, according to the experimental plan designed based on a central composite design including 12 central points (6 factors: concentration of the production medium, concentration of the feed medium, initial cell density, temperature, time to start adding the feed medium, pH after shifting). For all conditions, culture medium was sampled on day 13, 14, and 15 of culture (a total of 168 samples) and centrifuged (at 3000 rpm for 5 minutes). Supernatant was Protein-A purified, and then was used for measuring the ratio of Q-CDR-Clipped Variants.

### Analytical method

Viable cell number and viable cell ratio were measured by trypan blue staining. Q-CDR-Clipped Variants were detected as a peak by cation exchange high-performance liquid chromatography using a cation column (ProPac WCX-10).

### Results

Results are shown in Fig. 5 and Fig. 6. The ratio of Q-CDR-Clipped Variants was affected by both temperature and pH after shifting. Within the range tested (temperature of from 36 to 38°C and pH after shifting from 6.70 to 7.10), the ratio of Q-CDR-Clipped Variants was reduced more in culturing at 36°C and culturing at shifted pH of 7.10. There was an interaction between temperature and pH after shifting (shifted pH). It was found that the ratio of Q-CDR-Clipped Variants can be reduced even under the culturing condition at 38°C, when the shifted pH was lowered to 6.70.

Q-CDR-Clipped Variants were successfully controlled to 4% or less, by controlling culture temperature to 36-38°C and pH on or after day 3 of culture to 6.70-7.10.

### [Example 8] Removal of Q-CDR-Clipped Variants by cation exchange chromatography

Utilizing the difference in electrostatic property of Q-CDR-Clipped Variants and antibody Emicizumab, a method for separating these was established. An example is described below.

A column was filled with Capto SP ImpRes (GE) or a permissible substitute therefor as a cation exchange resin and equilibrated. The column was loaded with a solution of antibody Emicizumab containing Q-CDR-Clipped Variants to let them both absorbed. After loading, the column was washed with a phosphate buffer solution containing sodium chloride, and then, only the variants on acidic side including Q-CDR-Clipped Variants were specifically eluted so that they were separated and removed from antibody Emicizumab. In order to show how well the removal went, the washed fraction, the eluted fraction, and the loaded fraction before separation were CE-HPLC analyzed (Fig. 7).

The conditions of the loading, washing, and eluting were as shown below.

Loading: a Tris-hydrochloric acid buffer solution containing antibody Emicizumab adjusted at pH 5.0 was loaded on the condition of 33 g of antibody Emicizumab per 1 L of resin.

Washing: a phosphate buffer solution containing 25 mmol/L sodium chloride adjusted at pH 7.2 was passed through the 3.5 CV column at room temperature.

Eluting: a phosphate buffer solution containing 100 mmol/L sodium chloride adjusted at pH 6.5 was passed through the 6.5 CV column at room temperature.

### [Example 9] Analysis on molecular structure of the Protected Disulfide Isoforms

Antibody preparations for Emicizumab and a Protected Disulfide Isoform were prepared (antibody at a concentration of 7.54 mg/mL, 150 mmol/L arginine, 20 mmol/L histidine-aspartic acid, pH 6.0). SAXS measurement was carried out using line-collimated X-ray beam (Cu Kα, λ = 0.1542 nm) generated with the SAXSess mc2 system (Anton Paar, Graz, Austria). Temperature for measurement was set at 25°C. Two-dimensional imaging plates were used for the detection. Exposure time for the X-ray beam was set to 30 minutes. The two-dimensional scattered intensity was transformed to a single-dimensional scattered intensity I(q), on the SAXSQuant software (Anton Paar). Here, q is a scattering vector and is defined as q = (4π/λ)sin(θ/2) (θ is a scattering angle). Scattering curves were normalized against scattered intensity at q = 0 for the beam transmitted through the beam stopper, and then were processed for blank correction (with buffer and capillary) and optical system (desmearing) correction. Guinier plotting was carried out on the corrected scattering curves under the conditions satisfying q x Rg < 1.3, to obtain radius of gyration, Rg (nm); however, when there was a drop of scattered intensity in a small angle side, data for the range of q corresponding thereto was excluded in carrying out Guinier plotting, so as to avoid effects by interparticle repulsion. In a system assuming that there was no interparticle interaction (structure factor S(q) = 1), scattered intensity I(q) is given as a Fourier transform of pair distance distribution function p(r). By applying the indirect Fourier transform method (NPL 9) to the corrected scattering curves, p(r) for the particles were obtained. The maximum dimension Dmax (nm) was obtained from the x-intercept of p(r). The measurement was carried out three times for each of Emicizumab antibody preparation (Main) and the Protected Disulfide Isoform antibody preparation (BiAb3).

As a result, the Protected Disulfide Isoform had the average Rg value of 4.8 nm or less (the value 0.3 nm or more smaller than that of Emicizumab) and the average Dmax value of 16.5 nm or less (the value 1.4 nm or more smaller than that of Emicizumab), and exhibited 6% or more smaller average Rg value and 7.5% or more smaller average Dmax value as compared to Emcizumab. It is reported that the Dmax values of IgG4 antibody molecules, the same subclass of antibodies as Emicizumab, correspond to the distance between the tips of the two Fab domains (NPL 10); therefore, it is considered that the distance between the tips of the two Fab domains of the Protected Disulfide Isoform was shortened, thereby giving smaller values of Rg, which represents a distance from the center of mass of a molecule. Based on the above, it was confirmed that the Protected Disulfide Isoform takes the molecular structure having a shorter distance between the N termini of the J-chain/Q-chain than Emicizumab (Fig. 8). For bispecific antibodies for mediating interaction between two types of antigens, the distance between the Fab domains should be of crucial importance in determining inter-antigen interactions in view of the three-dimensional structure. Therefore, how to control the percentage of such isoforms in a pharmaceutical composition is an important task, not only for Emicizumab but, generally for antibody pharmaceuticals comprising a bispecific antibody.

### [Example 10] Molecular structure analysis by HDX-MS (Hydrogen-Deuterium eXchange Mass Spectrometry)

Antibody preparations for Emicizumab and for each of the Protected Disulfide Isoforms were prepared (antibody at a concentration of 1mg/mL, 150 mmol/L arginine, 20 mmol/L histidine-aspartic acid, pH 6.0) and HDX-MS measurement (measurements at deuterium exchange times 30 s, 60 s, 120 s, 240 s, 480 s, 960 s, 1920 s, and 3840 s) was carried out using the HDX-MS device (Orbitrap Fusion Lumos (Thermo Fisher Scientific), UltiMate30000RSLCnano (Thermo Fisher Scientific) with HDX-PAL (LEAP Technologies)).

As a result, prominent difference in deuterium exchange rate (%D) in the HDX-MS measurement was observed in the peptide comprising the amino acid residues from position 146 according to EU numbering of the Q chain to position 174 according to EU numbering of the Q chain (the 152^{nd} position to the 180^{th} position from the N terminus side of SEQ ID NO: 10) and the peptide comprising the amino acid residues from position 146 according to EU numbering of the J chain to position 174 according to EU numbering of the J chain (the 148^{th} position to the 176^{th} position from the N terminus side of
SEQ ID NO: 11). On the basis of this result, it was confirmed that the Protected Disulfide Isoforms have a different structure in these regions as compared with Emicizumab (Fig. 9).

### [Industrial Applicability]

The antibody variants and isoforms of the present invention have extremely reduced FVIII mimetic activity as compared with Emicizumab; therefore, the pharmaceutical compositions of the present invention comprising Emicizumab and having lowered content ratio for such antibody variants and isoforms are useful as a means for treating hemophilia. The methods for analyzing the antibody variants and isoforms of the present invention are useful for evaluating quality of Emicizumab preparations, and also are useful in development of Emicizumab preparations with a lowered content ratio for the antibody variants and isoforms or in development of methods for suppressing formation of the antibody variants and isoforms.
Furthermore, the present invention relates to the following items:
1. A variant of an antibody that comprises a variable region comprising the amino acid sequence SISPSGQSTYYRREVKG (SEQ ID NO: 2), wherein
   (a) the amino acid residue R at the 12th position from the N terminus side of the sequence; or
   (b) the amino acid residues YYR at the 10th to 12th position from the N terminus side of the sequence,
   is deleted and the variable region is cleaved at the deletion site.
2. The antibody variant of item 1, wherein the sequence is a CDR sequence.
3. The antibody variant of item 1, wherein the sequence is a CDR2 sequence.
4. The antibody variant of item 1, wherein the sequence is a sequence comprised in a heavy chain.
5. The antibody variant of item 1, which is a variant of a bispecific antibody.
6. The antibody variant of item 1, which is a variant of Emicizumab.
7. A method for detecting the antibody variant of any one of items 1 to 6, comprising the step of separating a sample containing an antibody that comprises a variable region comprising the amino acid sequence SISPSGQSTYYRREVKG (SEQ ID NO: 2) by affinity chromatography, ion exchange chromatography, normal phase chromatography, reverse phase chromatography, hydrophilic interaction chromatography (HILIC), hydrophobic interaction chromatography (HIC), separation based on charge, size exclusion chromatography (SEC), gel permeation chromatography (GPC), or combinations thereof.
8. The method for detecting of item 7, which uses the antibody variant of any one of items 1 to 6 as a reference standard.
9. A pharmaceutical composition comprising the antibody variant of any one of items 1 to 6, wherein the percentage of the antibody variant in the total antibody molecules in the pharmaceutical composition is 5% or less.
10. The pharmaceutical composition of item 9, wherein the antibody is Emicizumab.
11. The pharmaceutical composition of item 9, which is obtained by a purification process comprising purification by cation exchange chromatography (CEX).
12. A method for suppressing production of the antibody variant of any one of items 1 to 6, comprising the step of culturing antibody producing cells at a pH of 7.1 or more, and/or at a culture temperature of 36°C or less.
13. An isoform of a bispecific antibody comprising a first heavy chain and a second heavy chain, wherein
   disulfide bonds are formed in the following:
   (1a) between cysteine at position 144 according to EU numbering of the first heavy chain and cysteine at position 200 according to EU numbering of the second heavy chain; and
   (1b) between cysteine at position 200 according to EU numbering of the first heavy chain and cysteine at position 144 according to EU numbering of the second heavy chain, or wherein
   disulfide bonds are formed in the following:
   (2a) between cysteine at position 226 according to EU numbering of the first heavy chain and cysteine at position 229 according to EU numbering of the second heavy chain; and
   (2b) between cysteine at position 229 according to EU numbering of the first heavy chain and cysteine at position 226 according to EU numbering of the second heavy chain.
14. The bispecific antibody isoform of item 13, wherein disulfide bonds are formed in (1a) and (1b).
15. An isoform of a bispecific antibody comprising a first heavy chain and a second heavy chain, characterized in that it elutes at a region more to the alkaline side than the bispecific antibody when separated using cation exchange chromatography.
16. The bispecific antibody isoform of any one of items 13 to 15, which is an isoform of Emicizumab.
17. A method for detecting the antibody isoform of any one of items 13 to 16, comprising the step of separating a sample containing a bispecific antibody by affinity chromatography, ion exchange chromatography, normal phase chromatography, reverse phase chromatography, hydrophilic interaction chromatography (HILIC), hydrophobic interaction chromatography (HIC), separation based on charge, size exclusion chromatography (SEC), gel permeation chromatography (GPC), or combinations thereof.
18. The method for detecting of item 17, which uses the antibody isoform of any one of items 13 to 16 as a reference standard.
19. A pharmaceutical composition comprising the bispecific antibody isoform of any one of items 13 to 16, wherein the percentage of the antibody isoform in the total antibody molecules in the pharmaceutical composition is 2% or less.
20. A method for reducing the content percentage of the bispecific antibody isoform of any one of items 13 to 16, comprising the step of purification by cation exchange chromatography.
21. The antibody isoform or variant of item 1, 13, or 15, wherein the biological activity of the antibody is markedly reduced.

## Claims

1. A variant of an antibody that comprises a variable region comprising the amino acid sequence SISPSGQSTYYRREVKG (SEQ ID NO: 2), wherein
(a) the amino acid residue R at the 12th position from the N terminus side of the sequence; or
(b) the amino acid residues YYR at the 10th to 12th position from the N terminus side of the sequence,
is deleted and the variable region is cleaved at the deletion site.

2. The antibody variant of claim 1, wherein the sequence is (i) a CDR sequence, (ii) a CDR2 sequence or (iii) a sequence comprised in a heavy chain.

3. The antibody variant of claim 1, which is a variant of a bispecific antibody.

4. The antibody variant of claim 1, which is a variant of Emicizumab.

5. A pharmaceutical composition comprising the antibody variant of any one of claims 1 to 4, wherein the percentage of the antibody variant in the total antibody molecules in the pharmaceutical composition is 5% or less.

6. The pharmaceutical composition of claim 5, wherein the antibody is Emicizumab.

7. The pharmaceutical composition of claim 5, which is obtained by a purification process comprising purification by cation exchange chromatography (CEX).

8. A method for suppressing production of the antibody variant of any one of claims 1 to 4, comprising the step of culturing antibody producing cells at a pH of 7.1 or more, and/or at a culture temperature of 36°C or less.

9. An isoform of a bispecific antibody comprising a first heavy chain and a second heavy chain, wherein
disulfide bonds are formed in the following:
(1a) between cysteine at position 144 according to EU numbering of the first heavy chain and cysteine at position 200 according to EU numbering of the second heavy chain; and
(1b) between cysteine at position 200 according to EU numbering of the first heavy chain and cysteine at position 144 according to EU numbering of the second heavy chain, or wherein
disulfide bonds are formed in the following:
(2a) between cysteine at position 226 according to EU numbering of the first heavy chain and cysteine at position 229 according to EU numbering of the second heavy chain; and
(2b) between cysteine at position 229 according to EU numbering of the first heavy chain and cysteine at position 226 according to EU numbering of the second heavy chain.

10. The bispecific antibody isoform of claim 9, wherein disulfide bonds of (1a) and (1b) are formed.

11. An isoform of a bispecific antibody comprising a first heavy chain and a second heavy chain, **characterized in that** it elutes at a region more to the alkaline side than the bispecific antibody when separated using cation exchange chromatography.

12. The bispecific antibody isoform of any one of claims 9 to 11, which is an isoform of Emicizumab.

13. A method for detecting the antibody isoform of any one of claims 9 to 12, comprising the step of separating a sample containing a bispecific antibody by affinity chromatography, ion exchange chromatography, normal phase chromatography, reverse phase chromatography, hydrophilic interaction chromatography (HILIC), hydrophobic interaction chromatography (HIC), separation based on charge, size exclusion chromatography (SEC), gel permeation chromatography (GPC), or combinations thereof.

14. The method for detecting of claim 13, which uses the antibody isoform of any one of claims 9 to 12 as a reference standard.

15. A pharmaceutical composition comprising the bispecific antibody isoform of any one of claims 9 to 12, wherein the percentage of the antibody isoform in the total antibody molecules in the pharmaceutical composition is 2% or less.

16. A method for reducing the content percentage of the bispecific antibody isoform of any one of claims 9 to 12, comprising the step of purification by cation exchange chromatography.

17. The antibody isoform or variant of claim 1, 9, or 11, wherein the biological activity of the antibody is markedly reduced.
